# EUROPEAN PATENT APPLICATION

(11) **EP 2 075 231 A1**
(43) Date of publication of application: **01.07.2009**
(21) Application number: 07380400.7
(22) Date of filing: 28.12.2007
(51) Int. Cl.: C01B 25/32, C05B 17/00, A61K 8/19, A61L 27/12, A23L 1/304

(54) **Biphasic calcium phosphate and method for obtaining same from fish bones**

(71) Applicant: Universidad de Vigo, E-36310 Vigo (ES)
(72) Inventor: Boutinguiza Larosi, Mohamed, 36310 Vigo (ES); Pou Saracho, Juan Maria, 36310 Vigo (ES); Comesana Pineiro, Rafael, 36310 Vigo (ES); Lusquinos Rodriguez, Fernando, 36310 Vigo (ES); Leon Fong, Betty Mireya, 36310 Vigo (ES)
(74) Representative: ABG Patentes, S.L.

(57) **Abstract**

The present invention relates to a biphasic calcium phosphate comprising between 5 and 40% by weight of beta-tricalcium phosphate; between 0.1 and 0.6% by weight of Mg; between 0.2 and 0.8% by weight of Na; between 0.1 and 0.5% by weight of Sr; between 0.01 and 0.1% by weight of K; the remaining part being hydroxylapatite, to processes for obtaining same as well as to its use as a biocompatible material, particularly as a bone replacement material.

## Description

### Field of the Invention

The present invention is comprised within the process for obtaining natural biphasic calcium phosphate with a suitable ratio between hydroxylapatite (Ca₁₀(PO₄)₆(OH)₂) and beta-tricalcium phosphate (Ca₃(PO₄)₂). It specifically relates to a controlled heat process using fish bones as a source or precursor material of said calcium phosphate.

### Background of the Invention

The bones and teeth of vertebrates are hybrid materials formed by mineral phases (65% of their weight), an organic matrix (25%) and the remaining 10% of water. The main component of the inorganic part of bone is hydroxylapatite, Ca₁₀(PO₄)₆(OH)₂. The inorganic part of bones has a specific crystalline structure, with a crystal morphology, size and orientation differentiating it from synthetic apatites. Biological apathies are deficient in calcium and have carbonates in their structure; they have low crystallinity with nanometric-sized crystals. Their small size and low crystallinity are factors justifying their solubility and constant bone regeneration by means of continuous dissolution and crystallization cycles. These properties make their behavior as a biocompatible material be better than that of synthetic apatites.

The currently most extended source of biological apatites is bovine bone. The first applications of these products as a bone replacement material date from the 1950s. After several decades of research, commercial materials lacking immunogenicity have been achieved, given that they are subjected to pyrolytic processes eliminating all the protein and cell elements that usually occupy the intertrabecular spaces of the bone, thus achieving a matrix that is very similar to the human bone matrix although its use is reduced to filling bone defects, due to its low mechanical strength (Pasquier G., Hardouin P., Fontaine C., Migaud H., Duquennoy A. "Les différents modes de comblement oseeux en chirurgie orthopédique". Rev. Rhum Mal. Ostéoartic, 59(12), 821-828. 1992). The use of bovine bone for these purposes has currently been affected by the appearance of the spongiform encephalopathy disease ("mad cow disease") due to the rejection caused by products derived from bovine cattle in potential patients.

Natural sources of calcium phosphates also include those with a marine origin, some of which have been the object of study for obtaining hydroxylapatite for medical purposes. Another type of hydroxylapatite with an organic origin and porous structure can be obtained from coral, since some types of scleractinia-type coral from the Porites genus (Porites goniopora) form a shell made up of parallel channels intercommunicated by micropores. This exoskeleton is formed by calcium carbonate, which is converted into hydroxylapatite upon causing a chemical exchange with phosphates in a water medium and in the presence of heat. In this process, the microscopic structure is conserved and allows the connective tissue and the bone in which it is implanted to grow towards the inside of the material. The greatest drawback of these products lies in the protected species condition that coral has in many countries, which limits its exploitation on an industrial level.

Another source of calcium carbonates is formed by some algae varieties, from which another type of natural microscopic hydroxylapatite derived from phycogenic algae is prepared. Algae can be classified according to their lamina, the most visible part conferring to them the appearance that they adopt, calcified algae being able to accumulate calcium carbonate crystals, either in calcite or aragonite form, providing them with a hard appearance and forming the precursor material for preparing hydroxylapatite. The lack of continuity of sources of raw material for obtaining these calcium phosphates limits the industrial exploitation of this type of materials with a biological origin.

Patent US2005/191226 has recently disclosed a method for preparing hydroxylapatite powder by means of heating fish scales to remove the organic part and collecting the remaining inorganic powder. Porous hydroxylapatite can be obtained from a sintering process. However, the low proportion of calcium phosphate obtained per kilo of processes scales also makes the industrial exploitation of this source of calcium phosphate with a biological origin difficult.

In view of the drawbacks existing in the state of the art, it is necessary to develop a process which allows obtaining natural calcium phosphate with a suitable proportion of hydroxylapatite, such that it can give rise to a biocompatible material, which can be particularly used as a bone replacement material.

### Brief Description of the Invention

The authors of the present invention have achieved producing biphasic calcium phosphate powder with a biological origin from fish bones as a precursor material, with a proportion of hydroxylapatite and beta-tricalcium phosphate allowing its use as a biocompatible material. Due to its natural origin and to the proportions of the different components, its use further extends to the food field, specifically as a food additive, and even as a component of formulations aimed at the treatment of osteoporosis.

The developed process further allows simply obtaining biphasic calcium phosphate in large amounts from fishing activity waste, it can therefore be easily implemented on an industrial scale. The enormous amount of waste produced daily by fishing activities makes the raw material very abundant, inexpensive and fully assured throughout the year.

Thus, one aspect of the present invention is formed by a biphasic calcium phosphate characterized in that it comprises:
- between 5 and 40% by weight of beta-tricalcium phosphate;
- between 0.1 and 0.6% by weight of Mg;
- between 0.2 and 0.8% by weight of Na;
- between 0.1 and 0.5% by weight of Sr;
- between 0.01 and 0.1% by weight of K;
- the remaining part being hydroxylapatite.

A second aspect of the invention is aimed at a process for preparing biphasic calcium phosphate as defined above, which comprises calcining fish bones.

In a particular aspect, the calcination is carried out a temperature comprised between 600 and 1200°C.

In another particular aspect of the invention, the calcination is carried out by means of applying a laser beam.

Another aspect of the invention is aimed at a biphasic calcium phosphate which can be obtained according to a process such as the one described above.

In another aspect, the invention relates to a food composition comprising biphasic calcium phosphate as defined above.

In another additional aspect, the present invention is aimed at a pharmaceutical composition comprising biphasic calcium phosphate as defined above.

In another aspect, the invention relates to the use of biphasic calcium phosphate as defined above as a slow-acting fertilizer for acid soil.

An additional aspect of the present invention is formed by the use of biphasic calcium phosphate as defined above as a food additive, particularly as an additive in multivitamin complexes, as a texturizing agent for flours and batters and breading, as an anti-caking agent in flours, milk powder, lyophilized products and soup powders.

In another aspect, the invention relates to the use of biphasic calcium phosphate as defined above as an additive for toothpaste.

In another aspect, the invention relates to the use of biphasic calcium phosphate as defined above as a biomaterial, particularly as a bone replacement material.

A final aspect relates to the use of biphasic calcium phosphate as defined above for manufacturing a pharmaceutical composition for the treatment and/or prevention of osteoporosis.

### Detailed Description of the Invention

In one aspect, the present invention is aimed at a biphasic calcium phosphate comprising between 5 and 40% by weight of beta-tricalcium phosphate; between 0.1 and 0.6% by weight of Mg; between 0.2 and 0.8% by weight of Na; between 0.1 and 0.5% by weight of Sr; between 0.01 and 0.1% by weight of K; the remaining part being hydroxylapatite.

In the context of the present invention, the term 'biphasic calcium phosphate' relates to a calcium phosphate having two differentiated phase, one phase corresponding to hydroxylapatite and another phase corresponding to beta-tricalcium phosphate.

The term 'hydroxylapatite' (also called HA) is understood as an apatite mineral, specifically a calcium phosphate having the following chemical formula: Ca₁₀(PO₄)₆(OH)₂. It is essentially a calcium phosphate including hydroxide with a Ca:P ratio of 1.67:1 and crystallizing in the monoclinic or hexagonal system.

'Beta-tricalcium phosphate' (also called beta-TCP) in turn relates to a calcium phosphate of molecular formula β-Ca₃(PO₄)₂, with a Ca:P ratio of 1.5:1 and it crystallizes in the rhombohedral system.

### Process for preparing biphasic calcium phosphate

The process for obtaining biphasic calcium phosphate object of the present invention comprises calcining fish bones. For the purpose of obtaining separate and dry bones, before their calcination, a series of previous steps for treating the fishing activity waste are carried out. Any process which allows separating bones could be applied, nevertheless, in one particular aspect of the invention the waste is treated according to the steps detailed below.

### 1) Separating the bones

The waste used will generally have a varying amount of soft tissues adhered to the bones, it is therefore necessary to first separate the bones from said soft tissues. This separation can be carried out by different methods. One possible method is cooking the waste until achieving a suitable detachment of said soft tissues from the bones. This cooking process can be carried out at about 100°C and atmospheric pressure or in other temperature and pressure conditions which allow speeding up the process. For example, it is possible to shorten the time necessary for achieving the detachment of the waste by increasing the pressure in the cooking vessel. Another method for separating the bones consists of subjecting the fish waste to a treatment by means of chemical agents such as soda. If the soft tissues are not strongly adhered to the bones, the separation can be carried out by mechanical methods such as the use of a high-speed water jet impacting on the fish waste.

### 2) Washing the bones

The next step is a washing with a water jet fed at overpressure to achieve spraying the soft tissue detached from the bones and entraining them, whereby achieving a complete separation of the bones.

### 3) Drying the bones

The bones are then subjected to a drying process to remove the excess water. This step can be carried out in an oven, microwave oven or in a drying chamber.

Once the bines are dry, they can optionally be subjected to a grinding process such that a powder is obtained.

After the mentioned steps, the bones as such or in their ground form, already separated and dry, are subjected to the calcinations process.

### Calcination

After separating and drying the bones, both in the ground and non-ground form, they are subjected to a heat process for their calcinations. All the organic matter present in the bones is thus removed, obtaining the inorganic part thereof.

In one particular aspect of the present invention, the calcination step is carried out at a temperature greater than 500°C, preferably a temperature comprised between 600°C and 1200°C, more preferably between 900 and 950°C.

The calcination temperature is preferably reached by means of a heating gradient comprised between 18 and 22°C/min, preferably of approximately 20°C/min.

A calcination time greater than 5 hours is required for temperatures close to 600°C, such time being shortened for higher temperatures, specifically about one hour for 1200 °C.

A biphasic calcium phosphate containing an amount of beta-tricalcium phosphate between 5 and 40% by weight, the remaining part being hydroxylapatite, is obtained by means of a suitable control of the calcination conditions. In one particular embodiment, a biphasic calcium phosphate with an amount of beta-tricalcium phosphate between 5 and 20% by weight is obtained.

The described calcination process can be carried out in a kiln.

In another particular aspect of the invention, the calcination step is carried out by means of applying a laser beam. In one variant of this process, when the bones have not been ground, they are subjected to a temperature of about 200-400°C, in a kiln for example, and at the same time they are irradiated by means of a laser beam. The high energy density reached by the laser beam on the bones makes the process much faster than with a conventional calcination. The calcination time can thus be shortened from 2-3 hours to about 10 minutes.

In another variant of the process, the bones can be directly irradiated with the laser beam without needing additional heating by other means. Nevertheless, in this case, to obtain homogeneous results in all the material, the bones must be ground before their calcination. The bone powder is spread on a surface (moving or stationary with respect to the laser beam) and this bone powder is scanned by means of the action of the laser beam focused thereon.

The laser beam can come from laser equipment with any wavelength such as, for example, a CO₂, CO, N₂, Nd:YAG, Er:YAG, Nd:glass, Yb:YAG; Ruby, HeNe, HeCd, HeHg, Cu, I, Ar, Kr laser, a laser diode, a fiber laser, a disk laser, a chemical laser, an excimer laser, an alexandrite laser, an emerald laser or a dye laser. In a preferred aspect, the laser beam comes from CO₂, Nd:YAG or fiber lasers.

The power necessary for these types of lasers can be between 30 and 1000 W, the best results having been obtained when working with a power between 100 and 500 W.

### Grinding

The calcined material can be subjected to a grinding process for the purpose of obtaining a product with a granulometry that is as homogenous as possible. This process can be carried out in a ball mill or any other similar method.

The fish bones used as a precursor material for obtaining calcium phosphate of the present invention can from any fish species. Nevertheless, in a particular embodiment, said bones can come from a species selected from pollock (pollachius pollachius), tuna (thunnus thunnus), cod (gadus morhua), red sea bream (pagellus bogaraveo), bonito (sarda sarda), gilt-head sea bream (sparus aurata), coal fish (pollachius virens), halibut (hippoglossus hippoglossus), horse mackerel (trauchurus trauchurus), sole (solea vulgaris), blue whiting (campogramma glaycos), bass (dicentrarchus labrax), hake (merluccius merluccius), dusky grouper (epinephelus marginatus), pompano (trachinotus ovatus), swordfish (xiphias gladius), monkfish (lophius piscatorius), turbot (scophthalmus maximus), pink cusk-eel (genypterus blacodes), blue shark (prionace glauca) and trout (salmo trutta).

The biphasic calcium phosphate which can be obtained according to the process described above forms an additional aspect of the present invention.

### Uses

In another particular aspect, the invention is aimed at the use of biphasic calcium phosphate as it has been defined above as a slow-acting fertilizer for acid soil. The different degree of water-solubility of the major components of biphasic calcium phosphate (beta-TCP greater than HA) makes phosphorus be released in a sustained manner over time.

In an additional aspect, the present invention relates to the use of biphasic calcium phosphate as it has been defined above as a food additive, particularly as an additive in multivitamin complexes, as a texturizing agent in flours and batters and breading, as an anti-caking agent in flours, milk powder, lyophilized products and soup powders.

The biphasic calcium phosphate of the present invention can further act as a soft polish for tooth enamel, as well as a tooth remineralizing agent. Therefore, in another aspect, the invention is aimed at the use of biphasic calcium phosphate as an additive for toothpaste.

In an additional aspect, the invention relates to the use of biphasic calcium phosphate as it has been defined above as a biomaterial, particularly as a bone replacement material. This application is especially relevant for repairing cranio- and maxillofacial defects, periodontal defects, bone fractures and other dental and orthopedic defects. In one particular embodiment, for its application as a biomaterial, the biphasic calcium phosphate of the invention is formulated in a suitable composition in an effective amount together with one or more additional components. Pharmacologically active agents, proteins, polysaccharides, other biocompatible polymers or the like are especially interesting among said additional components. The proteins involved in the skeleton structure such as the different forms of collagen, fibrin, fibrinogen, keratin, tubulin, elastin and the like, or structural polysaccharides, such as chitin, are particularly interesting. Pharmacologically active agents can include medicinal products increasing bone growth, useful as cell growth factors, or acting as antiinflammatory or anti-microbial agents.

Alginate, agar-agar gum and chitosan are particularly relevant among biocompatible polymers. These polymers increase the duration of the coagulation process and allow, where appropriate, adapting this duration to a suitable value. These compounds further confer rheological sliding properties to the composition which allow it to circulate along the conduits or through the openings, facilitating its injection on the application point.

These compositions can be prepared by means of conventional methods, such as by means of mixing all the dry components together with an aqueous phase at room temperature, for example. The mixing processes can include ball mixing, Brabender mixing, stirring between one or more rollers and a flexible container or the like. The mixing must preferably be complete and take place for a time which allows obtaining a uniform dispersion of the ingredients.

In addition to water, the aqueous phase can include other pharmacologically acceptable water-miscible liquids, particularly alkanols, more particularly polyols such as ethylene glycol, propylene glycol or glycerin. Various solutes such as hydroxides, acetates, phosphates or carbonates of alkali metals can likewise be included. The aforementioned biocompatible polymers can also be incorporated in said aqueous phase.

These compositions are particularly useful for carrying out a filling or restoration on a dental or surgical area. The mixture between the aqueous phase and the solid components provides a paste which can then be placed on the dental or bone area to assure its hardening at about 37°C. The composition can also be implanted by means of a syringe or catheter injection on the affected area.

In another aspect, the invention relates to the use of biphasic calcium phosphate as it has been defined above for manufacturing a pharmaceutical composition aimed at the treatment and/or prevention of osteoporosis.

In one particular embodiment of the invention, for its administration in the prevention and/or treatment of osteoporosis, the biphasic calcium phosphate of the invention is formulated in a suitable pharmaceutical composition, in the therapeutically effective amount, together with one or more active ingredients, in addition to pharmaceutically acceptable carriers, adjuvants or excipients.

The pharmaceutical composition is administered orally, either in solid or liquid form. Illustrative example of dosage forms for oral administration include tablets, capsules, granulates, solutions, suspensions, etc., and can contain conventional excipients such as binders, diluents, disintegrants, lubricants, wetting agents, etc., and can be prepared by conventional methods.

In another particular embodiment, for its administration in the prevention and/or treatment of osteoporosis, the biphasic calcium phosphate of the invention is formulated in a suitable food composition or a dietary supplement, comprising biphasic calcium phosphate and a food carrier. For the purposes of the present invention, food carrier is understood as any product which can be used in human or animal food or fits into the definition of foodstuff according to the European legislation in force. Persons skilled in the art can choose the suitable food carrier for each case from the conventional food carriers existing in the state of the art.

Said food composition can be in soluble powder form, can be a liquid concentrate, a snack or a ready-to-use formulation suitable for oral consumption or enteral administration. Examples of these compositions can be, among others, a dairy product or derivative thereof such as a shake, milk in general (including whole, semi-skimmed, skimmed, concentrated, pasteurized, aromatized, fermented milk, soybean milk, optionally supplemented with sugars, other carbohydrates, fats and other nutritional additives), a yogurt, etc.; a juice; a flour product or derivative thereof such as a cake, a bread, a biscuit; etc.

In another preferred embodiment, the food composition is a dietary supplement. Said dietary supplement can be an ingestible liquid composition such as a syrup or a beverage, or an ingestible solid composition, such as a bar, capsule or powder which can be reconstituted.

The amount of biphasic calcium phosphate in the food composition or in the dietary supplement which can be ingested by a patient will depend on a number of factors such as the condition of the patient, his or her body weight, age, among others. However, the suitable amount must be prescribed by a specialist and will be adjusted according to the aforementioned variables.

Biphasic calcium phosphate can in turn be used together with other additional products or drugs useful in the prevention and/or treatment of osteoporosis. Said additional products or drugs can form part of the same pharmaceutical composition, food composition or dietary supplement or, alternatively, they can be provided in the form of a separate composition for its simultaneous or non-simultaneous administration to that of the pharmaceutical composition, food composition or dietary supplement comprising biphasic calcium phosphate.

The following examples intend to illustrate the invention but must not be considered as limiting the scope thereof.

### Examples

### Example 1. Preparation of biphasic calcium phosphate from swordfish waste

Frozen swordfish waste was subjected to the processes described in the method object of the present invention, i.e.: cooking at 100°C for 1 hour, washing in an overpressurized water jet, drying in an oven, calcining in a kiln at 950 °C for 12 hours and grinding in a ball mill for one minute. The obtained product is a crystalline calcium phosphate having two phases: hydroxylapatite and beta-tricalcium phosphate in a proportion of approximately 79 and 20% respectively, and a Mg content of 0.2%, a Na content of 0.3%, a Sr content of 0.4% and a K content of 0.02%, all the proportions being by weight.

### Example 2. Preparation of biphasic calcium phosphate from blue shark waste

Frozen blue shark waste was subjected to the processes described in the method object of the present invention, i.e.: cooking at 100°C for 1 hour, washing in an overpressurized water jet, drying in an oven, calcination by means of a laser and grinding in a ball mill for one minute. The calcinations process by means of a laser consisted of subjecting the blue shark bones to a scan with a defocused laser beam at 70 W for approximately 10 minutes, resulting in a crystalline compound formed by hydroxylapatite and beta-tricalcium phosphate in a proportion of 94 and 5% respectively, and a Mg content of 0.2%, a Na content of 0.4%, a Sr content of 0.3% and a K content of 0.03%, all the proportions being by weight.

## Claims

1. A biphasic calcium phosphate **characterized in that** it comprises:
- between 5 and 40% by weight of beta-tricalcium phosphate;
- between 0.1 and 0.6% by weight of Mg;
- between 0.2 and 0.8% by weight of Na;
- between 0.1 and 0.5% by weight of Sr;
- between 0.01 and 0.1% by weight of K;
- the remaining part being hydroxylapatite.

2. A biphasic calcium phosphate according to claim 1, wherein the proportion by weight of beta-tricalcium phosphate is comprised between 5 and 20%.

3. A process for preparing biphasic calcium phosphate as defined in claims 1 or 2, comprising calcining fish bones.

4. A process according to claim 3, further comprising the previous step of grinding the fish bones.

5. A process according to claim 3, wherein the calcination is carried out at a temperature comprised between 600 and 1200°C, preferably between 900 and 950°C.

6. A process according to claim 5, wherein the temperature is reached by means of a heating gradient comprised between 18 and 22°C/min, preferably of approximately 20°C/min.

7. A process according to any of claims 3 to 6, wherein the calcination is carried out in a kiln.

8. A process according to claim 3 or 4, wherein the calcination is carried out by means of applying a laser beam.

9. A process according to claim 8, wherein when the laser beam is applied on fish bones that are not ground, the latter are further subjected at the same time to a temperature comprised between 200 and 400°C.

10. A process according to claims 8 and 9, wherein the power of the laser beam is comprised between 30 and 1000 W, preferably between 100 and 500 W.

11. A process according to any of claims 8 to 10, wherein the laser beam comes from a CO₂, CO, N₂, Nd:YAG, Er:YAG, Nd:glass, Yb:YAG; Ruby, HeNe, HeCd, HeHg, Cu, I, Ar, Kr laser, a laser diode, a fiber laser, a disk laser, a chemical laser, an excimer laser, an alexandrite laser, an emerald laser or a dye laser, preferably from a CO₂, Nd:YAG or fiber laser.

12. A process according to any of claims 3 to 11, wherein the fish bone comes from a species selected from pollock (pollachius pollachius), tuna (thunnus thunnus), cod (gadus morhua), red sea bream (pagellus bogaraveo), bonito (sarda sarda), gilt-head sea bream (sparus aurata), coal fish (pollachius virens), halibut (hippoglossus hippoglossus), horse mackerel (trauchurus trauchurus), sole (solea vulgaris), blue whiting (campogramma glaycos), bass (dicentrarchus labrax), hake (merluccius merluccius), dusky grouper (epinephelus marginatus), pompano (trachinotus ovatus), swordfish (xiphias gladius), monkfish (lophius piscatorius), turbot (scophthalmus maximus), pink cusk-eel (genypterus blacodes), blue shark (prionace glauca) and trout (salmo trutta).

13. A biphasic calcium phosphate obtainable according to a process such as that defined in any of claims 3 to 12.

14. A food composition comprising biphasic calcium phosphate as it is defined in any of claims 1 or 2.

15. A pharmaceutical composition comprising biphasic calcium phosphate as it is defined in any of claims 1 or 2.

16. The use of biphasic calcium phosphate as it is defined in claims 1 or 2 as a slow-acting fertilizer for acid soil.

17. The use of biphasic calcium phosphate as it is defined in claims 1 or 2 as a food additive, particularly as an additive in multivitamin complexes, as a texturizing agent for flours and batters and breading, as an anti-caking agent in flours, milk powder, lyophilized products and soup powders.

18. The use of biphasic calcium phosphate as it is defined in claims 1 or 2 as an additive for toothpaste.

19. The use of biphasic calcium phosphate as it is defined in claims 1 or 2 as a biomaterial, particularly as a bone replacement material.

20. The use of biphasic calcium phosphate as it is defined in claims 1 or 2 for manufacturing a pharmaceutical composition for the treatment and/or prevention of osteoporosis.
